# EUROPÄISCHE PATENTSCHRIFT

(11) **EP 2 454 988 B1**
(45) Veröffentlichungstag und Bekanntmachung des Hinweises auf die Patenterteilung: **01.05.2013**
(21) Anmeldenummer: 10014779.2
(22) Anmeldetag: 19.11.2010
(51) Int. Cl.: A61B 3/024

(54) **Vorrichtung und Verfahren zur Gesichtsfeldbestimmung**
Method and device for determining field of vision
Procédé et dispositif destinés à la détermination des zones du visage

(43) Veröffentlichungstag der Anmeldung: 23.05.2012
(73) Patentinhaber: Ziemer Ophthalmic Systems AG, 2562 Port (CH)
(72) Erfinder: Rathjen, Christian, 28197 Bremen (DE)
(74) Vertreter: Rentsch Partner AG

(56) Entgegenhaltungen:
- EP-A2- 0 045 897
- DE-A1- 19 538 382
- DE-U- 1 963 161
- US-B2- 7 309 128

## Beschreibung

### Technisches Gebiet

Die vorliegende Erfindung betrifft eine Vorrichtung und ein Verfahren zur Gesichtsfeldbestimmung eines Patienten. Die vorliegende Erfindung betrifft insbesondere eine Vorrichtung und ein Verfahren zur Bestimmung des visuellen Gesichtsfelds eines Patienten, basierend auf Blickrichtungen des Patienten, die beim Anzeigen von Testmustern auf einem Bildschirm bestimmt werden.

### Stand der Technik

Vorrichtungen zur Bestimmung des Gesichtsfelds eines Patienten werden als Perimeter bezeichnet. Perimeter werden insbesondere bei einem Glaukom (grüner Star) eingesetzt, um Gesichtfeldausfälle zu erfassen. Bei statischer Perimetrie fixiert der Patient in der Regel ein zentrales Fixiertarget und drückt auf einen Knopf, sobald er einen punktförmigen Lichtstimulus in der Peripherie sieht. Bei der kinetischen Perimetrie wird der Lichtstimulus bewegt. Messdaten sind, der Ort und die Lichtstärke des Reizes. Auf der Basis mehrerer solcher Punktprojektionen wird das Gesichtsfeld in Form einer Gesichtsfeldkarte erstellt.

US 7,309,128 beschreibt ein Verfahren und eine Vorrichtung zur Messung des visuellen Gesichtsfelds, in welchen dem Patienten computergesteuert Fixier- und Testbilder präsentiert werden. Die Fixierbilder werden dem Patienten auf zwei Anzeigen für jedes Auge separat dargestellt. Gemäss US 7,309,128 werden separate Spiegel verwendet, um die Fixierbilder separat auf die beiden Augen zu lenken. Das Testbild zur Gesichtsfeldbestimmung wird dem Patienten auf einem senkrecht zu den Sehachsen des Patienten angeordneten Bildschirm dargestellt. Um den Bereich zwischen Punkten, die gesehenen und nicht gesehenen Testbildern entsprechen, möglichst genau zu bestimmen, testet der Computer automatisch zusätzliche Punkte in diesem Bereich. US 7,309,128 verwendet zudem einen so genannten Eyetracker mit einem Bildsensor, beispielsweise ein lichtempfindlicher Sensor oder Sensor-Array oder eine CCD-Kamera (Charge Coupled Device), zum Erfassen von Bildern der Augen.

DE 2 427 733 beschreibt ein Gerät zur Gesichtsfeldbestimmung, welches drei Seitenwände aufweist, die dem Gerät einen etwa dreiecksförmigen Grundriss verleihen. Eine der Seitenwände enthält einen Bildschirm auf dem eine fixe und eine manuell bewegbare Testmarke angezeigt werden können. Eine weitere Seitenwand enthält ein Binokular, durch welches der Patient den Bildschirm über einen Spiegel sehen kann, der auf der dritten Seitenwand angebracht ist. Aufgrund der dreiecksförmigen Anordnung der Seitenwände haben die Augen des Patienten eine unterschiedliche Distanz zum Spiegel, doch DE 2 427 733 führt als Vorteile an, dass die untersuchende Person den Patienten von der Seite sehen und beurteilen kann, ob der Patient aufmerksam genug ist, und dass der Abstand des Patienten von den Testmarken durch den Spiegel wesentlich vergrössert wird, ohne dass dazu ein übermässig grosses Gerät erforderlich ist. Die manuelle Positionierung von Testmarken und die subjektive Erfassung des visuellen Gesichtsfelds liefern jedoch in der Regel nicht sehr genaue Resultate.

EP 0 045 897 beschreibt ein Gerät für Augenuntersuchungen, in welchem ein drehbar gelagerter Spiegel zum Falten von optischen Pfaden vorgesehen ist, über welche Testlichtbilder den Augen zugelenkt werden.

### Darstellung der Erfindung

Es ist eine Aufgabe der vorliegenden Erfindung eine Vorrichtung und ein Verfahren zur Gesichtsfeldbestimmung eines Patienten vorzuschlagen, welche mindestens gewisse Nachteile des Stands der Technik überwinden. Es ist insbesondere eine Aufgabe der vorliegenden Erfindung eine Vorrichtung und ein Verfahren zur Gesichtsfeldbestimmung eines Patienten vorzuschlagen, welche eine kompakte Vorrichtungsausführung und einen mobilen Einsatz ermöglichen.

Gemäss der vorliegenden Erfindung werden diese Ziele durch die Elemente der unabhängigen Ansprüche erreicht. Weitere vorteilhafte Ausführungsformen gehen ausserdem aus den abhängigen Ansprüchen und der Beschreibung hervor.

Eine Vorrichtung zur Gesichtsfeldbestimmung umfasst einen Bildschirm zum Anzeigen von Testmustern, ein Augenüberwachungsmodul zum Erfassen von Augenbewegungen des Patienten beim Betrachten des Bildschirms, und ein Verarbeitungsmodul, das eingerichtet ist, beim Anzeigen der Testmuster basierend auf den Augenbewegungen Blickrichtungen des Patienten zu bestimmen, und das Gesichtsfeld des Patienten basierend auf den für die Testmuster bestimmten Blickrichtungen zu ermitteln.

Die oben genannten Ziele werden durch die vorliegende Erfindung insbesondere dadurch erreicht, dass die Vorrichtung zur Gesichtsfeldbestimmung zudem einen zur Gesichtsfeldbestimmung parallel zur Bipupillarlinie des Patienten ausgerichteten Spiegel zum Umlenken der Testmuster vom Bildschirm zu den Augen umfasst. Dabei ist die Bipupillarlinie eine Gerade durch die Mitte beider Pupillen, wobei zur Gesichtsfeldbestimmung eine aufrechte Haltung des Patienten und damit eine horizontale Ausrichtung der Bipupillarlinie vorausgesetzt und angenommen wird. Durch die Verwendung des parallel zur Bipupillarlinie des Patienten ausgerichteten Spiegels ergibt sich eine besonders kompakte Ausführung der Vorrichtung zur Gesichtsfeldbestimmung, welche für beide Augen die selben geometrischen und optischen Messbedingungen ermöglicht.

Vorzugsweise ist der Bildschirm zur Gesichtsfeldbestimmung auf einer horizontalen Tragfläche liegend, mit von der Tragfläche abgewandter und im Wesentlichen horizontal ausgerichteter Bildschirmanzeigefläche positionierbar. Der Bildschirm ist vorzugsweise ein Flachbildschirm. Die horizontale Ausrichtung der Bildschirmanzeigefläche bei einer liegenden Positionierung des Bildschirms ermöglicht einen niedrigen Schwerpunkt und stabile Positionierung der Vorrichtung.

In einer Ausführungsform sind der Bildschirm und der Spiegel an einer ihrer Längsseiten um eine Achse drehbar so gekoppelt, dass eine spiegelnde Oberfläche des Spiegels und eine Bildschirmanzeigefläche des Bildschirms einander zugewandt auseinander- respektive zusammenklappbar sind. Durch das flexible Zusammenklappen wird ein einfacher Transport ermöglicht, was für Anwendungen im mobilen Einsatz beispielsweise in Altersheimen und Krankenhäusern von Vorteil ist. Der drehbare Spiegel ermöglicht dabei ein besonders einfaches Auf- und Abbauen der Vorrichtung für die Gesichtsfeldbestimmung.

In einer Ausführungsvariante umfasst die Vorrichtung einen Streulichtschutz mit zwei Streulichtschutzschirmen, die jeweils im Wesentlichen senkrecht zur oben angeführten Drehachse ausgerichtet sind. Die beiden Streulichtschutzschirme sind jeweils in einem Randbereich des Bildschirms und des Spiegels mit dem Bildschirm und dem Spiegel verbunden, und in einem Öffnungswinkel zwischen dem Bildschirm und dem Spiegel angeordnet. In einer zusammen- respektive aufklappbaren Variante ist der Streulichtschutz eingerichtet, sich im Öffnungswinkel zu öffnen, der sich beim Auseinanderklappen des Bildschirms und des Spiegels ergibt. In einer nicht zusammen- respektive aufklappbaren Variante ist der Streulichtschutz Teil eines statischen Gehäuses der Vorrichtung.

In einer weiteren Ausführungsvariante umfasst die Vorrichtung einen Sichtschirm, der zur Gesichtsfeldbestimmung so zwischen Bildschirmanzeigefläche und Patient positionierbar ist, dass die Bildschirmanzeigefläche vom Patienten nicht direkt sichtbar ist. Der Sichtschirm verhindert Verfälschungen der Gesichtsfeldbestimmung durch Lichtstimuli, die auf dem Bildschirm dargestellt und vom Patienten dort direkt wahrgenommen werden.

Die Vorrichtung umfasst einen Abstandssensor zur Bestimmung der relativen Distanz der Augen zum Spiegel und das Verarbeitungsmodul ist eingerichtet, das Gesichtsfeld des Patienten abhängig von der relativen Distanz der Augen zu ermitteln. Durch die dynamische Bestimmung und Berücksichtigung der Distanz der Augen vom Bildschirm sind unterschiedliche Stellungen des Spiegels beim Aufklappen der Vorrichtung möglich, ohne dass dadurch die Genauigkeit der Gesichtsfeldbestimmung merklich beeinflusst wird.

In einer weiteren Ausführungsvariante ist das Verarbeitungsmodul eingerichtet, eine Sequenz von Testmustern auf dem Bildschirm anzuzeigen, beim Anzeigen der Testmuster jeweils die Blickrichtung eines Auges des Patienten zu bestimmen und zugeordnet zum betreffenden Testmuster zu speichern, und das Gesichtsfeld des Patienten für eines oder beide Augen basierend auf den für die Sequenz der Testmuster erfassten Blickrichtungen zu ermitteln. Durch die automatische Erzeugung einer sequentiellen Abfolge von Testmustern und darauf basierten Ermittlung des Gesichtsfeld des Patienten wird eine besonders effiziente Gesichtsfeldbestimmung ermöglich, die insbesondere auch für eine selbständige Durchführung durch den Patienten geeignet ist. Das Verarbeitungsmodul ist beispielsweise eingerichtet, die Sequenz der Testmuster abhängig von Blickrichtungen, die für den Patienten erfasst werden, dynamisch anzupassen.

In einer Ausführungsvariante ist das Verarbeitungsmodul eingerichtet, eines oder mehrere Referenzmuster auf dem Bildschirm anzuzeigen. Das Augenüberwachungsmodul ist eingerichtet, Referenzmuster zu erfassen, die von einem Auge des Patienten reflektiert werden, und das Verarbeitungsmodul ist eingerichtet, die Blickrichtung eines Auges des Patienten jeweils basierend auf dem reflektierten Referenzmuster zu bestimmen.

In einer Ausführungsvariante ist das Verarbeitungsmodul eingerichtet, beim Anzeigen der Testmuster jeweils abhängig von den Augenbewegungen eine Reaktionszeit des Patienten zu erfassen, und im Gesichtsfeld des Patienten ortsabhängige Reaktionszeiten zu speichern.

In einer weiteren Ausführungsvariante ist das Augenüberwachungsmodul auf einer bei der Gesichtsfeldbestimmung vom Patienten abgewandten Seite des Spiegels angeordnet, und der Spiegel weist eine dem Augenüberwachungsmodul vorgelagerte, für mindestens gewisse Wellenlängen lichtdurchlässige Stelle zum Bestimmen der Augenbewegungen auf. In einer besonders einfachen Ausführung ist die lichtdurchlässige Stelle als Bohrung im Spiegel ausgestaltet.

In einer anderen Ausführungsvariante ist das Augenüberwachungsmodul auf einer bei der Gesichtsfeldbestimmung dem Patienten zugewandten Seite des Spiegels angeordnet, und das Augenüberwachungsmodul ist eingerichtet, die Augenbewegungen via den Spiegel zu bestimmen.

In einer anderen Ausführungsvariante ist das Augenüberwachungsmodul zur Gesichtsfeldbestimmung ausserhalb der spiegelnden Oberfläche des Spiegels mit direkter Sichtlinie auf die Augen des Patienten angeordnet.

In verschiedenen Ausführungsvarianten umfasst das Augenüberwachungsmodul eine oder zwei Bildsensoren und ist entsprechend eingerichtet, die Augenbewegungen eines Auges monoskopisch respektive stereoskopisch zu ermitteln.

In einer weiteren Ausführungsvariante ist der Spiegel zur Feldwinkelvergrösserung mit einer nicht-planen Spiegeloberfläche ausgestaltet, beispielsweise konkav und/oder segmentiert.

Neben der Vorrichtung zur Gesichtsfeldbestimmung betrifft die vorliegende Erfindung zudem ein Verfahren zur Gesichtsfeldbestimmung, in welchem Testmuster auf einem Bildschirm angezeigt werden, Augenbewegungen eines Patienten erfasst werden, Blickrichtungen des Patienten beim Anzeigen der Testmuster basierend auf den Augenbewegungen bestimmt werden, und das Gesichtsfeld des Patienten basierend auf den für die Testmuster bestimmten Blickrichtungen ermittelt werden, wobei die Testmuster vom Bildschirm über einen zur Gesichtsfeldbestimmung parallel zur Bipupillarlinie des Patienten ausgerichteten Spiegel zu den Augen umgelenkt werden.

### Kurze Beschreibung der Zeichnungen

Nachfolgend wird eine Ausführung der vorliegenden Erfindung anhand eines Beispieles beschrieben. Das Beispiel der Ausführung wird durch die folgenden beigelegten Figuren illustriert:
- Figur 1:: zeigt eine perspektivische und schematische Darstellung einer Vorrichtung zur Gesichtsfeldbestimmung, welche einen Bildschirm zur Darstellung von Testmustern und einen Spiegel zum Umlenken der Testmuster vom Bildschirm zu den Augen eines Patienten umfasst.
- Figur 1a:: zeigt eine perspektivische und schematische Darstellung einer Variante der Vorrichtung mit zwei Linsen für eine binokulare Gesichtsfeldbestimmung.
- Figur 2:: zeigt einen Querschnitt (unterer Teil) und eine Aufsicht (oberer Teil), welche schematisch die Ausrichtung des Spiegels parallel zur Bipupillarlinie des Patienten und die Umlenkung der Testmuster vom Bildschirm zu den Augen des Patienten via den Spiegel illustrieren.
- Figur 3a:: zeigt in einer Querschnittansicht schematisch eine zusammenklappbare Ausführung der Vorrichtung zur Gesichtsfeldbestimmung im geschlossenen, zusammengeklappten Zustand.
- Figuren 3b und 3c:: zeigen in der Querschnittansicht schematisch die zusammenklappbare Ausführung der Vorrichtung im aufgeklappten Zustand, wobei der Bildschirm unterschiedlich schräg zur horizontalen Tragfläche positioniert ist.
- Figur 4:: zeigt ein Flussdiagramm, welches ein Beispiel einer Sequenz von Schritten eines Verfahrens zur Bestimmung des Gesichtsfelds eines Patienten illustriert.

### Wege zur Ausführung der Erfindung

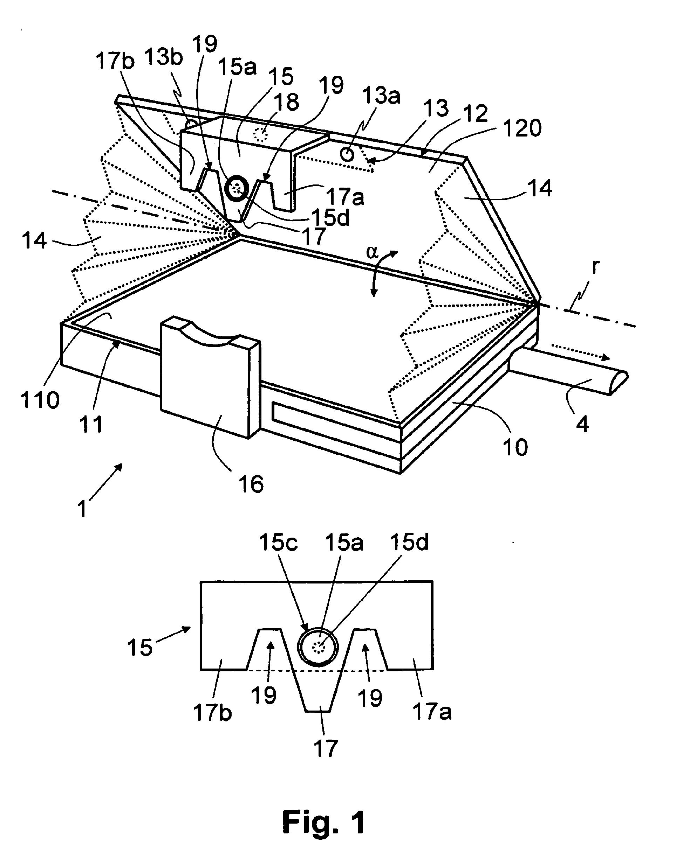

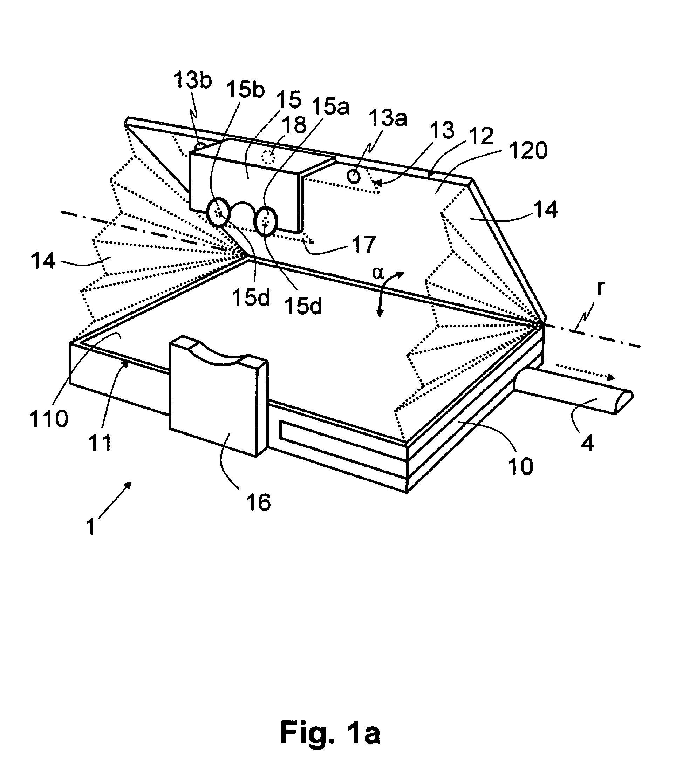

In den Figuren 1, 1a, 2, 3a, 3b und 3c bezieht sich das Bezugszeichen 1 auf eine ophthalmologische Vorrichtung zur Bestimmung des visuellen Gesichtsfelds eines Patienten 20.

Wie in den Figuren 1, 1a, 2, 3a, 3b und 3c schematisch dargestellt ist, umfasst die ophthalmologische Vorrichtung 1 einen Bildschirm 11 mit einer Bildschirmanzeigefläche 110, einen Spiegel 12 mit einer spiegelnden Oberfläche 120, ein Verarbeitungsmodul 10 und ein Augenüberwachungsmodul 13 (Eyetracker).

In einer Variante umfasst die Vorrichtung 1 einen optionalen Abstandssensor 18 zur Bestimmung der relativen Distanz der Augen 2 respektive des zu testenden Auges 2 des Patienten 20 zum Spiegel 12. Insbesondere bei stereoskopischen Ausführungen des Augenüberwachungsmoduls 13 ist der Abstandssensor 18 nicht hardwaremässig als Sensorelement sondern als Teil des Verarbeitungsmoduls 10 ausgeführt, das heisst Abstandswerte werden durch eine Abstandsfunktion des Verarbeitungsmoduls 10 auf der Basis der vom Augenüberwachungsmodul 13 erfassten Bilder ermittelt. Ein Abstandssensor 18 ist nicht notwendig, wenn die Position der Augen 2 durch eine Kopf- und/oder (optionale) Kinnstütze 15, 16 hinreichend genau festgelegt ist und der Abstand kalibriert ist.

Das Verarbeitungsmodul 10 ist mit dem Bildschirm 11, dem Augenüberwachungsmodul 13 und dem optionalen Abstandssensor 18 verbunden und umfasst einen oder mehrere betriebsfähige Prozessoren mit Daten- und Programmspeicher. Das Verarbeitungsmodul 10 umfasst programmierte Softwaremodule mit Programmcode zur Steuerung der Prozessoren, derart, dass diese Funktionen ausführen, welche nachfolgend detailliert beschrieben werden. Der Programmcode ist auf einem greifbaren computerlesbaren Medium gespeichert, das fest oder entfernbar mit den Prozessoren verbunden ist. Der Fachmann wird verstehen, dass die Funktionen des Verarbeitungsmoduls 10 in alternativen Ausführungsvarianten teilweise oder vollständig mit Hardwarekomponenten ausführbar sind.

Das Verarbeitungsmodul 10 ist im Wesentlichen eingerichtet, Testmuster für die Gesichtsfeldbestimmung zu generieren und auf dem Bildschirm 11 darzustellen, vom Augenüberwachungsmodul 13 erfasste Augenbewegungen des Patienten 20 entgegenzunehmen, zu speichern und auszuwerten, und vom optionalen Abstandssensor 18 die relative Distanz der Augen 2 des Patienten 20 zum Spiegel 12 entgegenzunehmen, zu speichern und auszuwerten.

In einer Ausführungsvariante umfasst die ophthalmologische Vorrichtung 1 eine oder mehrere einstellbare Linsen 15a, 15b, wie in Figuren 1, 1a schematisch dargestellt ist, deren Brennweiten manuell oder durch das Verarbeitungsmodul 10 automatisch einstellbar sind.

Der Bildschirm 11 ist als aktiver selbstleuchtender oder passiver, fremdbeleuchteter Flachbildschirm ausgestaltet. In einer Ausführungsvariante ist der Bildschirm 11 berührungsempfindlich ausgestaltet, als sogenannter Touch- oder Multitouch-Screen, und dient als Eingabeelement zur Eingabe von Patientendaten, Messparametern und Steuerbefehlen, etc. In einer alternativen Ausführungsvariante oder zusätzlich umfasst die Vorrichtung 1 eine drahtlose und/oder kontaktbasierte Datenschnittstelle für den Datenaustausch mit einem externen Personal Computer (PC), der zur Steuerung der Vorrichtung 1 und/oder für die Erfassung und Speicherung von Testresultaten eingesetzt wird. Vorzugsweise verfügt die ophthalmologische Vorrichtung 1 oder der externe PC über einen zur Vorrichtung 1 zusätzlichen externen Bildschirm zur Anzeige von Messresultaten und/oder eingegebenen Steuerbefehlen und Steuerparametern.

Wie in den Figuren 1, 1a, 2, 3a, 3b und 3c dargestellt ist, sind der Bildschirm 11 und der Spiegel 12 an einer ihrer Längsseiten entlang der Achse r miteinander verbunden. Vorzugsweise, aber nicht zwingend, sind der Bildschirm 11 und der Spiegel 12 über ein Drehgelenk, beispielsweise ein Scharnier, um die Drehachse r drehbar miteinander verbunden. Die drehbare Kopplung des Bildschirms 11 und des Spiegels 12 ermöglicht ein Zusammenklappen und Aufklappen der ophthalmologischen Vorrichtung 1, ähnlich wie bei einem Laptop-Computer. In der vorliegenden ophthalmologischen Vorrichtung 1 ist jedoch der Bildschirm 11 für eine im Wesentlichen horizontale, flach liegende Positionierung eingerichtet und bildet die Basis der Vorrichtung 1.

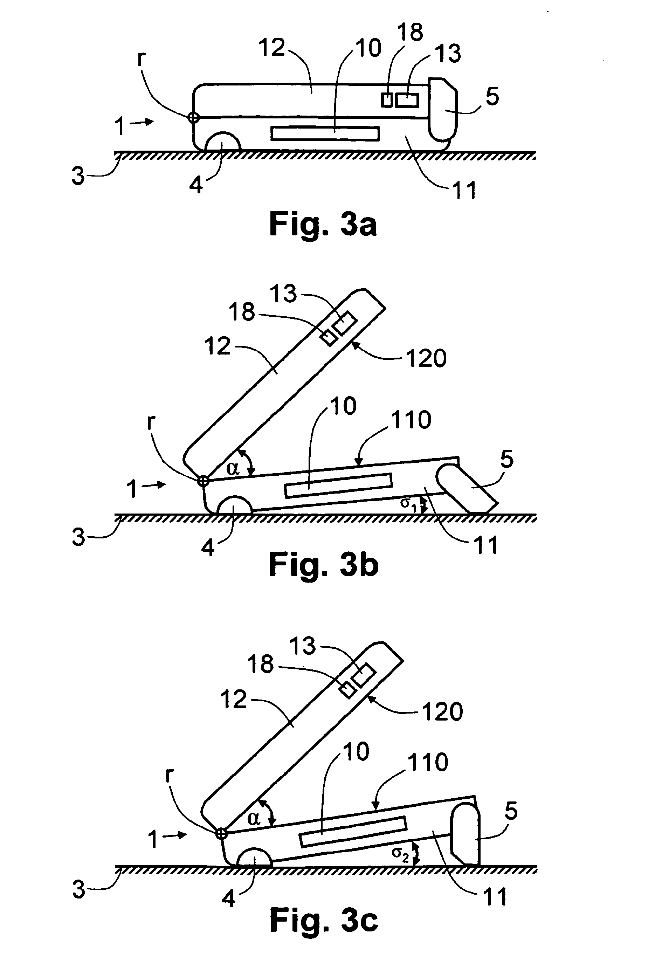

Im geschlossenen, zusammengeklappten Zustand, sind der Bildschirm 11 und der Spiegel 12 im Wesentlichen parallel zueinander ausgerichtet, wobei die Bildschirmanzeigefläche 110 und die spiegelnde Oberfläche 120 des Spiegels 12 sich einander zugeneigt gegenüberliegen, wie in der Figur 3a ersichtlich ist.

Im Betriebsmodus zur Gesichtsfeldbestimmung ist der Spiegel 12 aufgeklappt und es bildet sich ein Winkel α zwischen der Bildschirmanzeigefläche 110 und der spiegelnden Oberfläche 120 des Spiegels 12. Zur Gesichtsfeldbestimmung liegt der Winkel α beispielsweise im Bereich zwischen 30° und 60°, wobei bevorzugte Winkelpositionen dem Benutzer durch einen Rastermechanismus angezeigt werden.

Wie in den Figuren 3a bis 3c schematisch illustriert ist, umfasst die ophthalmologische Vorrichtung 1 in einer Ausführungsvariante einen drehbaren Traggriff 5, der auf der der Drehachse r gegenüberliegenden Längsseite des Bildschirms 11 angebracht ist und im Wesentlichen parallel zur Drehachse r verläuft. Der Traggriff 5 ist um eine parallel zur Drehachse r verlaufende Achse drehbar und so eingerichtet, dass der Bildschirm 11 und damit die Bildschirmanzeigefläche 110 durch eine entsprechende Stellung des Traggriffs 5 um einen wählbaren Winkel σ₁, σ₂ schräg zur horizontalen Tragfläche 3 positionierbar ist, wobei diese geneigte Stellung im Sinne der vorliegenden Erfindung noch immer als im Wesentlichen horizontale Ausrichtung des Bildschirms 11 respektive der Bildschirmanzeigefläche 110 zu betrachten ist, da der Winkel σ₁ , σ₂ beispielsweise 15° nicht übersteigt.

Eine Stabilisierung der durch den Bildschirm 11 gebildeten Basis der Vorrichtung 1 erfolgt durch die optionalen Stützfüsse 4, die einrastend um eine zur Drehachse r parallele horizontale Achse drehbar ausgestaltet sind. Die Stützfüsse 4 weisen beispielsweise einen halbkreisförmigen Querschnitt auf. In einer Variante sind die Stützfüsse 4 zudem seitlich aus dem Basiskörper des Bildschirms 11, entlang einer zur Drehachse r parallelen Achse herausziehbar, wie in den Figuren 1, 1a schematisch illustriert ist. Die Stützfüsse 4 dienen überdies oder alternativ als Griffe, an denen sich der Patient festhalten bzw. die Hände darauf abstützen kann.

In einer Variante umfasst die ophthalmologische Vorrichtung 1 als Streulichtschutz zudem zwei seitliche Streulichtschutzschirme 14, wie in den Figuren 1, 1a schematisch dargestellt ist. Die Streulichtschutzschirme 14 sind im Wesentlichen senkrecht zur Drehachse r ausgerichtet und jeweils in seitlichen Randbereichen am Bildschirm 11 und am Spiegel 12 angebracht. In der zusammen- und aufklappbaren Version der Vorrichtung 1 sind die Streulichtschutzschirme 14 flexibel ausgestaltet, beispielsweise gefaltet oder mit mehreren parallel angeordneten bewegbaren Scheibenelementen, ähnlich wie bei einer lrisblende, und eingerichtet sich beim Aufklappen des Spiegels 12 beidseitig im Öffnungswinkel α zwischen dem Bildschirm 11 und dem Spiegel 12 zu öffnen, ggf. durch Entfalten respektive Auseinanderschieben.

Wie in der Figuren 1, 1a ersichtlich ist, umfasst die ophthalmologische Vorrichtung 1 zudem einen, beispielsweise justierbaren, Sichtschirm 17, der zur Gesichtsfeldbestimmung so zwischen Bildschirmanzeigefläche 110 und die Augen 2 des Patienten positioniert oder positionierbar ist, beispielsweise durch entsprechendes Einschwenken respektive Drehen, dass die Bildschirmanzeigefläche 110 vom Patienten 20 nicht direkt einsehbar sondern nur über den Spiegel 12 sichtbar ist. Zusätzlich oder als Alternative zu einem Sichtschirm 17 wird mittels des Verarbeitungsmodul 10 basierend auf den vom Augenüberwachungsmodul 13 erfassten Augenbewegungen überprüft werden, ob eine Reaktion auf den richtigen Lichtstimulus erfolgt.

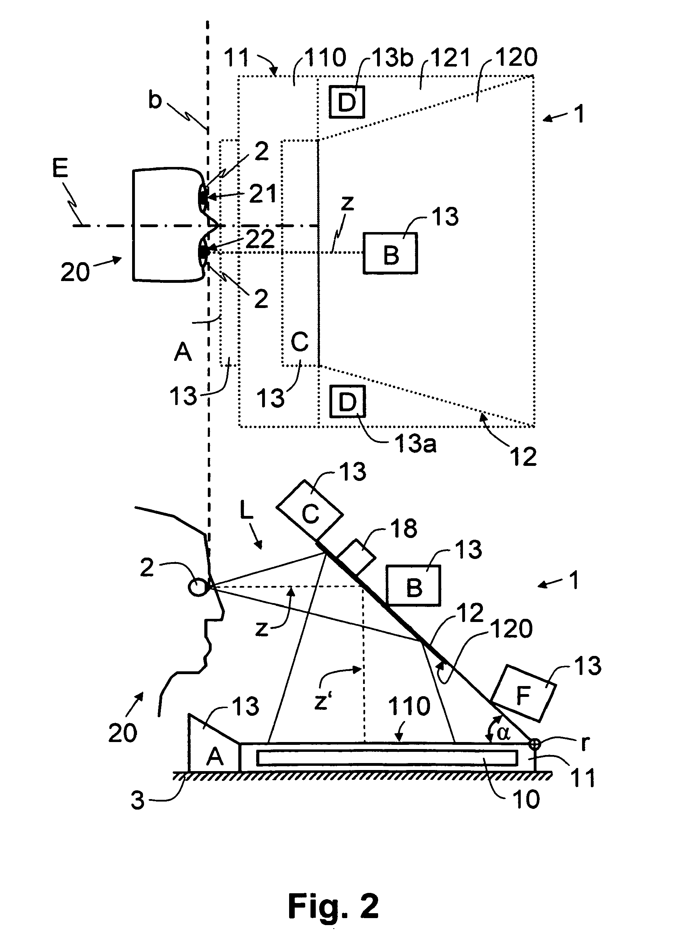

Die Figur 2 illustriert im unteren Teil schematisch im Querschnitt die Umlenkung der Lichtstrahlen L des auf dem Bildschirm 11 angezeigten Testmusters über den aufgeklappten Spiegel 12 zu den Augen 2 des Patienten 20. Die Figur 2 illustriert zudem die im Wesentlichen parallele Ausrichtung des Spiegels 12 zu der Bipupillarlinie b des Patienten 20, eine Gerade, die die Mitten der Pupillen 21, 22 des Patienten 20 miteinander verbindet, wie in der schematischen Aufsicht des Kopfs des Patienten 20 im oberen Teil der Figur 2 ersichtlich ist. In einer planen Ausgestaltung der Spiegeloberfläche 120 ist entsprechend auch die Spiegeloberfläche 120 im Wesentlichen parallel zur Bipupillarlinie b des Patienten 20 ausgerichtet. Der Bildschirm 11 respektive die Bildschirmanzeigefläche 110 ist vorzugsweise so angeordnet und ausgerichtet, dass ein entlang der Sehachse z projizierter und über den Spiegel 12 umgelenkter Strahl z' im Wesentlichen senkrecht auf die Bildschirmoberfläche 110 trifft, und somit sowohl der Bildschirm 11 respektive die Bildschirmanzeigefläche 110 als auch der Spiegel 12 respektive die Spiegeloberfläche 120 parallel zur Bipupillarlinie b angeordnet sind.

In einer alternativen Ausgestaltung ist die Spiegeloberfläche 120 zur Vergrösserung des Feldwinkels und damit einhergehenden ausgedehnteren Gesichtsfeldbestimmung konkav und/oder mit segmentierter Spiegeloberfläche 120 ausgestaltet. Auch bei einer derartig konkav respektive segmentiert ausgestalteten Spiegeloberfläche 120 ist die Ausrichtung des Spiegels 12 im Sinne der vorliegenden Erfindung noch immer als im Wesentlichen parallel zur Bipupillarlinie b des Patienten 20 ausgerichtet zu betrachten, da eine normal zur Spiegeloberfläche 120 ausgerichtete optische Achse bei der Gesichtsfeldbestimmung in der Symmetrieebene E verläuft, die zwischen den Augen 2 des Patienten 20 angeordnet senkrecht zur Bipupillarlinie b des Patienten 20 ausgerichtet ist.

In einer Variante ist das Verarbeitungsmodul 10 eingerichtet, die Brennweite der einstellbaren Linsen 15a, 15b bei Testmustern mit Lichtreflexmarken im äusseren Bereich des Gesichtfelds automatisch anzupassen, damit der Patient 20 auch diese scharf abgebildet sehen kann. Dies ist insbesondere bei der Ausführungsvariante mit konkav und/oder segmentiert ausgestalteter Spiegeloberfläche 120 von Vorteil, wo der Bereich des Gesichtfelds durch den erweiterten Feldwinkel vergrössert wird. In einer alternativen Ausgestaltung ist zu diesem Zeck auf oder oberhalb der Bildschirmanzeigefläche 110 eine Fresnel-Linse oder ein diffraktiv optisches Element angebracht.

Die Figur 2 illustriert zudem verschiedene Anordnungen des Augenüberwachungsmoduls 13. Das Augenüberwachungsmodul 13 umfasst in einer monoskopischen Ausführung einen Bildsensor (Kamera) und in einer stereoskopischen Ausführung zwei Bildsensoren (Kameras) 13a, 13b.

Das Bezugszeichen A bezieht sich auf eine Anordnung des Augenüberwachungsmoduls 13, in welcher eine oder zwei an der von der Drehachse r abgewandten Vorderseite des Bildschirms 11 angeordnete Bildsensoren (Kameras) die Augenbewegungen des Patienten 20 über den Spiegel 12 verfolgen und erfassen.

Die Bezugszeichen B und F beziehen sich auf Anordnungen, in welchen eine oder zwei Bildsensoren (Kameras) des Augenüberwachungsmoduls 13 auf der von der Spiegeloberfläche 120 abgewandten Rückseite des Spiegels 12 angeordnet sind und die Augenbewegungen des Patienten 20 durch eine lichtdurchlässige Stelle im Spiegel 12 verfolgen und erfassen. Die lichtdurchlässige Stelle ist beispielsweise als Bohrung oder als für gewisse Lichtwellenlängen durchlässige Beschichtung auf der Spiegeloberfläche 120 ausgeführt. In der mit dem Bezugszeichen B bezeichneten Anordnung, ist/sind die Bildsensoren (Kamera/s) des Augenüberwachungsmoduls 13 beispielsweise an der Stelle angeordnet, wo die Sehachse z bei entsprechender Einstellung des Winkels α den Spiegel 12 durchdringt. In der mit dem Bezugszeichen F bezeichneten Anordnung, ist/sind die Bildsensoren (Kamera/s) des Augenüberwachungsmoduls 13 unterhalb der Sehachse z angeordnet und erfasst/erfassen die Augenbewegungen des Patienten schräg von unten. Die mit F bezeichnete Anordnung hat den Vorteil, dass eine Erfassung der Augenbewegung von schräg unten in der Regel bessere, d.h. genauere und klarere, Resultate ergibt als Erfassungen von oberhalb der Sehachse z. Zudem sind für gewisse Augenüberwachungsmodule 13, insbesondere stereoskopische Ausführungen, Arbeitsabstände zu den Augen 2 notwendig, welche keine praktische Integration in die Vorrichtung 1, insbesondere in einen Träger des Spiegels 12 ermöglichen, wohingegen die mit F bezeichnete Anordnung grössere Arbeitsabstände und somit eine Integration in die Vorrichtung 1 respektive in den Träger des Spiegels 12 ermöglicht. Zudem ermöglicht die mit F bezeichnete Anordnung eine einfachere Ausgestaltung des Spiegels 12, da das Augenüberwachungsmodul 13 in einem Bereich des Spiegels 12 angeordnet werden kann, der für die Funktionstüchtigkeit der Vorrichtung 1 nicht zwingend eine spiegelnde Oberfläche 120 aufweisen muss, so dass der Spiegel 12 im Bereich des Augenüberwachungsmoduls 13 tatsächlich transparent ausgestaltet werden kann und nicht mit einer Bohrung versehen werden muss.

Die Bezugszeichen C und D beziehen sich auf Anordnungen, in welchen eine oder zwei Bildsensoren (Kameras) 13a, 13b des Augenüberwachungsmoduls 13 ausserhalb der spiegelnden Oberfläche 120 des Spiegels 12 angeordnet sind und die Augenbewegungen des Patienten 20 bei der Gesichtsfeldbestimmung in direkter Sichtlinie verfolgen und erfassen. In der mit C bezeichneten Anordnung ist das Augenüberwachungsmodul 13 an der von der Drehachse r abgewandten vorderen Seitenlinie des Spiegels 12 angeordnet. In der mit D bezeichneten Anordnung sind vorzugsweise zwei Bildsensoren (Kameras) 13a, 13b jeweils in einem seitlichen, vorzugsweise nicht spiegelnden Randbereich 121 des Spiegels 12, auf der dem Patienten 20 bei der Gesichtsfeldbestimmung zugewandten Seite des Spiegels 12 angeordnet.

In einer Variante umfasst das Verarbeitungsmodul 10 eine Kalibrierfunktion, welche eine geometrische und/oder radiometrische Kalibrierung der Vorrichtung 1 respektive des Verfahrens zur Gesichtsfeldbestimmung ermöglicht oder ausführt. Die Kalibrierfunktion wird beispielsweise auf der Basis von Messdaten durchgeführt, die von einem Bildsensor 15d, z.B. ein lichtempfindlicher Sensor oder Sensor-Array oder eine Kamera, erfasst werden. Der Bildsensor 15d ist beispielsweise fest oder entfernbar an der Stelle angebracht, wo das Auge des Patienten positioniert wird, z.B. an der Kopfstütze 15 in oder bei den Bohrungen 15c der Linsen 15a, 15b, beispielsweise wegschwenkbar. Die vom Bildsensor 15d erfassten Messdaten umfassen im Wesentlichen die vom Bildschirm 11 abgestrahlten und über den Spiegel 12 umgelenkten Kalibrierungs- oder Testbildmuster, aus welchen die Kalibrierfunktion Basiswerte zur Kalibrierung der Vorrichtung 1 respektive des Verfahrens zur Gesichtsfeldbestimmung erzeugt. Die Basiswerte umfassen beispielsweise örtliche Helligkeitswerte und Helligkeitsverteilungen der abgestrahlten und erfassten Muster und dadurch aktuelle Abstrahlungseigenschaften des Bildschirms 11 respektive der Bildschirmanzeigefläche 110, Positionierung der Muster und dadurch die relative Ausrichtung zu den Mustern, und/oder die Grösse und damit die Distanz zu den Mustern. Die bestimmten Basiswerte werden bei der Gesichtsfeldbestimmung berücksichtigt, beispielsweise indem sie in die Berechnung respektive Bestimmung von Schwellwerten einfliessen und/oder indem die Abstrahlung der Testmuster für die Gesichtsfeldbestimmung hinsichtlich örtlicher Helligkeit, Grösse und/oder Position der Darstellung auf der Bildschirmanzeigefläche 110 angepasst werden. In einer alternativen oder zusätzlichen Variante wird die örtliche Verteilung der Abstrahlungshelligkeit, also die radiometrischen Basiswerte des Bildschirms 11, mittels auf der Bildschirmanzeigefläche 110 aufgesetzter radiometrischer Sensoren erfasst.

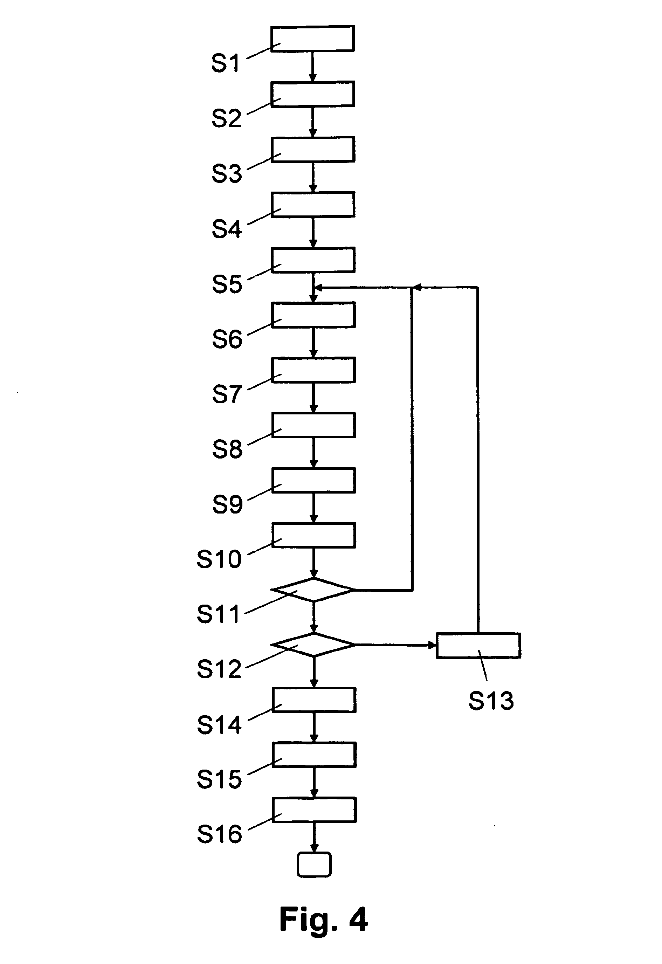

In den nachfolgenden Abschnitten werden ein Beispiel einer Sequenz von Schritten zur Ausführung des ophthalmologischen Verfahrens zur Bestimmung des visuellen Gesichtfelds eines Patienten 20 mittels der Vorrichtung 1, sowie die Funktionen des Verarbeitungsmoduls 10 mit Bezug zur Figur 4 beschrieben.

Typischerweise erfolgt die Gesichtsfeldbestimmung separat für jedes Auge 2 des Patienten, wobei das Verarbeitungsmodul 10 beispielsweise automatisch für das nicht betroffene Auge 2 eine nicht dargestellte Sichtblende schliesst, die z.B. in die Linse(n) 15a, 15b integriert oder vor- respektive nachgeschaltet ist.

Im Schritt S1 des ophthalmologischen Verfahrens wird die ophthalmologische Vorrichtung 1 eingeschaltet und in der zusammenklappbaren Version auf einer horizontalen Tragfläche 3 positioniert, aufgeklappt und gegebenenfalls die Stützfüsse 4 ausgezogen.

Im Schritt S2 wird über Eingabeelemente, beispielsweise über den berührungsempfindlichen Bildschirm 11, eine Tastatur oder Bedienknöpfe, die auszuführende Gesichtsfeldbestimmung ausgewählt, beispielsweise durch Wahl einer von mehreren definierten Sequenzen von Testmustern, und Patientendaten eingegeben, beispielsweise Identifizierungsdaten und Angaben zum Sehvermögen respektive zu refraktiven Korrekturerfordernissen.

Im Schritt S3 wird der Patient 20 zur Vorrichtung 1 positioniert, dabei wird das Kinn auf der Kinnstütze 16 positioniert und die Position der Linsen 15a, 15b auf die Augenhöhe angepasst, entweder durch Justieren der Höhe der Kinnstütze 16 oder durch Einstellen des Winkels α zwischen Spiegel 12 und Bildschirm 11, beispielsweise durch entsprechendes Positionieren der als Visier ausgebildeten Kopfstütze 15. Vorzugsweise erfolgt als Alternative oder zusätzlich eine Abstützung des Kopfs des Patienten auf respektive an der Kopfstütze 15 und dadurch eine relative Positionierung und Abstandsfixierung der Augen 2 in Bezug zur Vorrichtung 1 respektive zum Spiegel 12 und zur Bildschirmanzeigefläche 110. Wie in der Figur 1 ersichtlich ist und zusätzlich in einer Detailansicht dargestellt ist, weist das symmetrisch ausgestaltete Visier respektive die Kopfstütze 15 in einer Ausführungsvariante neben einer mit den Linsen 15a versehenen Bohrung 15c zwei jeweils beidseits der Bohrung 15c angeordnete Ausnehmungen 19 zur Positionierung der Nase des Patienten auf, je nachdem, ob das linke oder rechte Auge 2 untersucht werden soll. Dabei wird jeweils das nicht zu untersuchende Auge 2 durch einen der Lappen 17a, 17b des Visiers abgedeckt, während das zu untersuchende Auge 2 vor der Bohrung 15c mit den Linsen 15a positioniert ist und auf den Spiegel 12 blickt. In dieser Ausführung ist der Sichtschirm 17 unterhalb der Bohrung für die Linsen 15a, 15b zwischen den zwei Ausnehmungen 19 angeordnet. Der Sichtschirm 17 entspricht beispielsweise in Fläche und Form den Ausnehmungen 19, wie in der Detailansicht durch die gestrichelte Linie angedeutet wird. Zudem weist der Sichtschirm 17 in der dargestellten Ausführung mit den Ausnehmungen 19 jeweils eine gemeinsame Seitenkante auf, die im Bereich der Ausnehmungen 19 jeweils eine Innenkante und im Bereich des Sichtschirms 17 eine Aussenkante bilden. Auch die beiden Lappen 17a, 17b des Visiers dienen als Sichtschirm zum Vermeiden, dass die Bildschirmanzeigefläche 110 vom Patienten 20 nicht direkt einsehbar sondern nur über den Spiegel 12 sichtbar ist.

In einer alternativen Ausführung, beispielsweise als auswechselbares Visier, sind wie in Figur 1a dargestellt zwei Bohrung mit Linsen 15a, 15b vorgesehen, die wie bei einer Brille links respektive rechts von einer einzigen Ausnehmung 19 für die Nase angeordnet sind, so dass wahlweise beide Augen 2 gleichzeitig (binokular) oder auch nur eines der Augen 2 untersucht werden können, ohne dass dazu die Kopfposition respektive Nasenposition verschoben werden muss. Die Bipupillarlinie b des Patienten 20 wird im Wesentlichen parallel zur Drehachse r und damit zum Spiegel 12 und zum Bildschirm 11 ausgerichtet.

Im Schritt S4 werden allfällig notwendige Dioptriekorrekturen für den Patienten 20 durch manuelle oder durch das Verarbeitungsmodul 10 automatisch vorgenommene Einstellungen der Linsen 15a, 15b vorgenommen. Dabei wird dem Patienten 20 ein Fixiermuster und/oder Lesetestbilder auf dem Bildschirm 11 dargestellt und über den Spiegel 12 auf seine Augen 2 gelenkt. Weitere Feinabstimmungen der Ausrichtung von Spiegel 12 und Bildschirm 11 werden vorgenommen. Gegebenenfalls wird der Neigungswinkel σ₁, σ₂ des Bildschirms 11 durch geeignete Stellung des Traggriffs 5 justiert.

Nach erfolgreicher Positionierung, Ausrichtung und optischer Einstellung der ophthalmologischen Vorrichtung 1 zum Patienten 20 und dessen Augen 2, wird im Schritt S5 die gewählte Sequenz zur Gesichtsfeldbestimmung gestartet, beispielsweise auf Knopfdruck oder durch Berühren des Bildschirms 11. Dabei wird mittels des Abstandssensors 18 die aktuelle relative Distanz der Augen 2 des Patienten 20 zum Spiegel 12 respektive zur Bildschirmanzeigefläche 110 ermittelt und gespeichert. Als Alternative wird die Distanz aufgrund des aktuellen Winkels α zwischen Bildschirm 11 und Spiegel 12, und der definierten Geometrie der Vorrichtung 1 bestimmt.

Im Schritt S6 erzeugt das Verarbeitungsmodul 10 auf dem Bildschirm 11 das erste Testmuster der Sequenz zur Gesichtsfeldbestimmung. Das angezeigte Testmuster wird von der Bildschirmanzeigefläche 110 durch spiegelnde Reflexion auf der Spiegeloberfläche 120 des Spiegels 12 den Augen 2 des Patienten 20 zugeführt. Insbesondere bei einer Ausführung mit erweitertem Feldwinkel justiert das Verarbeitungsmodul 10 nötigenfalls die Brennweite der Linsen 15a, 15b, wenn das Textmuster Lichtstimuli im äusseren Feldwinkelbereich erzeugt.

Die Testmuster umfassen jeweils einen zur Gesichtfeldbestimmung an einem definierten Punkt angeordneten Lichtstimulus in unterschiedlicher Grösse und Helligkeit. Vorzugsweise umfassen die Testmuster neben dem eigentlichen Lichtstimulus zudem statische Fixiermuster, beispielsweise in Form von Fixier- oder Zielmarken, und Referenzmuster, beispielsweise Placidomuster, welche auf dem zu beobachtenden Auge 2 reflektiert und von den Augenüberwachungsmodulen 13 zur Bestimmung von Augenbewegungen und Blickrichtungen erfasst werden. Das Verarbeitungsmodul 10 ist zudem eingerichtet alternative Fixiermuster zu generieren und darzustellen, wenn die typischerweise zentral angeordnete Fixier- oder Zielmarke vom betreffenden Auge 2 des Patienten 20 aufgrund eine Sehdefekts nicht sehbar ist. In einer weiteren Variante umfasst die ophthalmologische Vorrichtung 1 externe Fixiertargets, die ausserhalb des Bereichs angeordnet sind, wo die Sehachse z bei entsprechender Einstellung des Winkels α den Spiegel 12 durchdringt, und die über einen teildurchlässigen Spiegel auf die Sehachse z eingespiegelt werden. Für andere, z.B. neurophysiologische Untersuchungen, ist die Kopfstütze 15 zweckmässigerweise entfernbar, und das Augenüberwachungsmodul 13 und das Verarbeitungsmodul 10 sind eingerichtet, beide Augen 2 (gleichzeitig) zu erfassen und zu tracken.

In einer weiteren Variante umfasst die ophthalmologische Vorrichtung 1 ein Keratometer zur Vermessung, Ermittlung und Speicherung der Hornhauttopologie des Patienten, vorzugsweise unter Zuhilfenahme der oben genannten Placidomuster und Bildsensoren (Kameras) 13a, 13b des Augenüberwachungsmoduls 13.

Im Schritt S7 bezieht das Verarbeitungsmodul 10 die vom Augenüberwachungsmodul 13 während der Darstellung des Testmusters erfassten Augenbewegungen. Dabei speichert das Verarbeitungsmodul 10 beispielsweise eine Sequenz von Bildern des zu vermessenden Auges 2.

Im Schritt S8 ermittelt das Verarbeitungsmodul 10 aufgrund der erfassten Augenbewegungen die Blickrichtung des Patienten 20 bei der Darstellung des Testmusters. Zudem ermittelt das Verarbeitungsmodul 10 aufgrund der vom Augenüberwachungsmodul 13 erfassten Bilddaten den relativen Abstand der Augen 2 in Bezug zur Vorrichtung respektive zum Spiegel 12, wenn der Abstand nicht a priori definiert ist, beispielsweise durch Kopf- und/oder Kinnstützen 15, 16. Dabei wertet das Verarbeitungsmodul 10 beispielsweise die gespeicherte Bildsequenz mit den erfassten Augenbewegungen basierend auf der Distanz der Augen 2 des Patienten 20 zum Spiegel 12 aus. Die Bildsequenz lässt eine genaue zeitliche und örtliche Zuordnung von Darstellung des Testmusters und Augenbewegungen des Patienten 20 zu.

Falls eine mit dem Testmuster zeitlich und örtlich korrelierende Augenbewegung des Patienten 20 in Richtung des Lichtstimulus des betreffenden Testmusters detektierbar ist, wird im Schritt S9 die Position des vom Patienten 20 im Testmuster wahrgenommenen Lichtstimulus als positiver Punkt in einer geometrischen Gesichtsfeldkarte für den Patienten 20 erfasst und gespeichert. In einer Variante wird andernfalls die betreffende Position als negativer Punkt ebenfalls erfasst. Somit wird mittels des Verarbeitungsmodul 10 basierend auf den vom Augenüberwachungsmodul 13 erfassten Augenbewegungen ermittelt und überprüft, ob eine Reaktion auf den Lichtstimulus erfolgt und ob eine Reaktion auf den richtigen Lichtstimulus erfolgt.

Im Schritt S10 ermittelt das Verarbeitungsmodul 10 bei einer positiven Reaktion auf das Testmuster die Reaktionszeit des Patienten 20 durch Ermitteln der Zeitdauer von der Darstellung des Testmusters bis zur detektierten korrelierenden Augenbewegung des Patienten 20 in Richtung des Lichtstimulus im betreffenden Testmuster. Dabei wertet das Verarbeitungsmodul 10 beispielsweise die gespeicherte Bildsequenz mit den erfassten Augenbewegungen aus. Die Reaktionszeit wird in der Gesichtsfeldkarte der erfassten Position des Lichtstimulus zugeordnet gespeichert.

Im Schritt S11 überprüft das Verarbeitungsmodul 10, ob die im Schritt S5 gestartete Testmustersequenz abgearbeitet wurde oder ob die Sequenz weitere darzustellende Testmuster mit Lichtstimuli in anderen Positionen umfasst. Falls weitere Testmuster anzuzeigen sind, fährt das Verarbeitungsmodul 10 mit dem Verfahren im Schritt S6 fort, andernfalls im Schritt S12.

Im Schritt S12 überprüft das Verarbeitungsmodul 10, ob in der erzeugten Gesichtsfeldkarte Grenzbereiche mit aneinandergrenzenden Zonen mit positiv respektive negativ markierten Punkten eine Auflösung mit höherer Messauflösung erfordern, beispielsweise im Vergleich zu einem definierten Genauigkeitsschwellwert, und/oder ob die Gesichtsfeldkarte Bereiche mit scheinbar widersprüchlichen positiven und negativen Punkten aufweist. Falls eine verfeinerte und/oder überprüfte Gesichtsfeldbestimmung erforderlich ist, fährt das Verarbeitungsmodul 10 mit dem Verfahren im Schritt S13 fort, andernfalls im Schritt S14. Dabei gilt es festzuhalten, dass die Helligkeit des Lichtstimulus variiert wird und für verschiedene Helligkeitsstufen unterschiedliche einzelne oder mehrere punktuelle positiv/negativ-Werte oder positiv/negativ-Karten erstellt werden, in welchen jeweils für eine der Helligkeitsstufe die im Testmuster wahrgenommenen respektive nicht wahrgenommenen Lichtstimuli als positive respektive negative Punkte dargestellt respektive gespeichert sind. Basierend auf den positiv/negativ-Karten verschiedener Helligkeitsstufen erstellt das Verarbeitungsmodul 10 eine als Empfindlichkeitskarte ausgestaltete Gesichtsfeldkarte, wobei die Empfindlichkeit beispielsweise in dB angegeben wird, oder für einen oder mehrere Punkte punktuelle Empfindlichkeitswerte oder partielle Gesichtsfeldkarten.

Im Schritt S13 erzeugt das Verarbeitungsmodul 10 für die im Schritt S11 bestimmten fraglichen Bereiche der Gesichtsfeldkarte zusätzliche Sequenzen mit Testmustern, die ein feineres Raster von Lichtstimuli in den verfeinert zu untersuchenden Bereichen aufweisen, und fährt mit dem Verfahren im Schritt S6 fort.

Im Schritt S14 speichert das Verarbeitungsmodul 10 die Messresultate, d.h. die erstellte Gesichtsfeldkarte respektive die punktuellen Empfindlichkeitswerte oder partiellen Gesichtsfeldkarten dem Patienten 20 zugeordnet mit Datum und Zeit. Die Messresultate umfassen beispielsweise eine Tabelle mit den positiven und gegebenenfalls negativen Positionen, die durch kartesische und/oder Polarkoordinaten bestimmt sind, und Reaktionszeiten, die den positiven Punkten zugeordnet sind. In einer Variante werden die Messresultate, insbesondere die partiellen oder vollständigen Gesichtsfeldkarten, als darstellbares Bild erzeugt, in welchem die positiven und gegebenenfalls negativen Positionen, Kontrast, Lumineszenz, Latenzzeiten, Reaktionszeiten und/oder Empfindlichkeitswerte hinsichtlich Helligkeit und/oder Farbe, also wellenlängenabhängig, visuell in einer zweidimensionalen Karte dargestellt sind, beispielsweise mit unterschiedlichen Farben, und Wertangaben, die numerisch oder in unterschiedlicher Helligkeit oder Farbintensität wiedergegeben sind.

Im Schritt S15 gibt das Verarbeitungsmodul 10 die Messresultate, beispielsweise die erstellte(n) Gesichtsfeldkarte(n), als Darstellung auf dem Bildschirm 11 und/oder als Ausdruck auf einem mit der Vorrichtung 1 verbundenen Drucker aus.

Im optionalen Schritt S16 übermittelt das Verarbeitungsmodul 10 die Messresultate, beispielsweise die erstellte(n) Gesichtsfeldkarte(n), als digitale Datei über eine Kommunikationsverbindung an ein Kommunikationsendgerät, zum Beispiel an einen zuständigen Augenarzt und/oder eine Patientendatenbank, beispielsweise mittels eines Kommunikationsmoduls über eine Geräteschnittstelle, z.B. kontaktlos via Bluetooth oder kontaktbehaftet via USB, ein Kommunikationsnetz, z.B. drahtlos über ein WLAN oder ein mobiles Funktelefonnetz, oder verdrahtet über ein LAN, WAN, ISDN, POTS oder ein anderes Kommunikationsnetz einschliesslich des Internets.

In weiteren Ausführungsvarianten, ist das Verarbeitungsmodul 10 eingerichtet, die ophthalmologische Vorrichtung 1 zur Durchführung von Lese- und/oder Sehtests zu steuern, in welchen die Seh- und Lesefähigkeit sowie Seh- und Lesegeschwindigkeit ermittelt wird.

Schliesslich soll hier auch festgehalten werden, dass das Verarbeitungsmodul 10 in einer Variante eingerichtet ist, verschiedene vom Benutzer wählbare und konfigurierbare gespeicherte Messprogramme mit unterschiedlichen Teststrategien durchzuführen, welche unterschiedliche Schwellwertstrategien für die Ermittlung der Empfindlichkeiten und/oder verschiedene Messpunktraster aufweisen. Solche Strategien können (anwendungsabhängig) zum Beispiel zur Reduzierung der Messzeit oder zur besseren Eingrenzung von Bereichen mit Gesichtfeldausfällen dienen.

Schliesslich soll hier angeführt werden, dass in der Beschreibung zwar Computerprogrammcode spezifischen funktionalen Modulen zugeordnet wurde und dass die Ausführung von Schritten in einer bestimmten Reihenfolge dargestellt wurde, dass der Fachmann jedoch verstehen wird, dass der Computerprogrammcode unterschiedlich strukturiert und die Reihenfolge von mindestens gewissen Schritten geändert werden kann, ohne dabei vom Schutzgegenstand abzuweichen.

## Patentansprüche

1. Vorrichtung (1) zur Gesichtsfeldbestimmung, umfassend:
einen Bildschirm (11) mit einer Bildschirmanzeigefläche (110) zum Anzeigen von Testmustern,
ein Augenüberwachungsmodul (13) zum Erfassen von Augenbewegungen eines Patienten (20), und
ein Verarbeitungsmodul (10), das eingerichtet ist, beim Anzeigen der Testmuster basierend auf den Augenbewegungen Blickrichtungen des Patienten (20) zu bestimmen, und das Gesichtsfeld des Patienten (20) basierend auf den für die Testmuster bestimmten Blickrichtungen zu ermitteln,
**gekennzeichnet durch** einen zur Gesichtsfeldbestimmung parallel zur Bipupillarlinie (b) des Patienten (20) ausgerichteten Spiegel (12) zum Umlenken der Testmuster vom Bildschirm (11) zu den Augen (2), und
**dadurch**, dass die Vorrichtung (1) einen Abstandssensor (18) zur Bestimmung der relativen Distanz der Augen (2) zum Spiegel (12) respektive zur Bildschirmanzeigefläche (110) umfasst, und
dass das Verarbeitungsmodul (10) eingerichtet ist, das Gesichtsfeld des Patienten (20) abhängig von der relativen Distanz der Augen (2) zu ermitteln.

2. Vorrichtung (1) nach Anspruch 1, **dadurch gekennzeichnet, dass** der Bildschirm (11) zur Gesichtsfeldbestimmung auf einer horizontalen Tragfläche (3) liegend, mit von der Tragfläche (3) abgewandter und im Wesentlichen horizontal ausgerichteter Bildschirmanzeigefläche (110) positionierbar ist.

3. Vorrichtung (1) nach einem der Ansprüche 1 oder 2, **dadurch gekennzeichnet, dass** der Bildschirm (11) und der Spiegel (12) an einer ihrer Längsseiten um eine Achse (r) drehbar so gekoppelt sind, dass eine spiegelnde Oberfläche (120) des Spiegels (12) und eine Bildschirmanzeigefläche (110) des Bildschirms (11) einander zugewandt auseinander- respektive zusammenklappbar sind.

4. Vorrichtung (1) nach einem der Ansprüche 1 bis 3, **gekennzeichnet durch** einen Streulichtschutz mit zwei Streulichtschutzschirmen (14), die jeweils im Wesentlichen senkrecht zur Achse (r) ausgerichtet in einem Randbereich des Bildschirms (11) und des Spiegels (12) mit dem Bildschirm (11) und dem Spiegel (12) verbunden sind, und in einem Öffnungswinkel (α) zwischen dem Bildschirm (11) und dem Spiegel (12) angeordnet sind.

5. Vorrichtung (1) nach einem der Ansprüche 1 bis 4, **dadurch gekennzeichnet, dass** die Vorrichtung (1) einen Sichtschirm (17) umfasst, der zur Gesichtsfeldbestimmung so zwischen Bildschirmanzeigefläche (110) und Patient positionierbar ist, dass die Bildschirmanzeigefläche (110) vom Patienten (20) nicht direkt sichtbar ist.

6. Vorrichtung (1) nach einem der Ansprüche 1 bis 5, **dadurch gekennzeichnet, dass** das Verarbeitungsmodul (10) eingerichtet ist, eine Sequenz von Testmustern auf dem Bildschirm (11) anzuzeigen, beim Anzeigen der Testmuster jeweils die Blickrichtung eines Auges (2) des Patienten (20) zu bestimmen und zugeordnet zum betreffenden Testmuster zu speichern, und das Gesichtsfeld des Patienten (20) für eines oder beide Augen (2) basierend auf den für die Sequenz der Testmuster erfassten Blickrichtungen zu ermitteln.

7. Vorrichtung (1) nach Anspruch 6, **dadurch gekennzeichnet, dass** das Verarbeitungsmodul (10) eingerichtet ist, die Sequenz der Testmuster abhängig von Blickrichtungen, die für den Patienten (20) erfasst werden, dynamisch anzupassen.

8. Vorrichtung (1) nach einem der Ansprüche 1 bis 7, **dadurch gekennzeichnet, dass** das Verarbeitungsmodul (10) eingerichtet ist, eines oder mehrere Referenzmuster auf dem Bildschirm (11) anzuzeigen, dass das Augenüberwachungsmodul (13) eingerichtet ist, von einem Auge des Patienten (20) reflektierte Referenzmuster zu erfassen, und dass das Verarbeitungsmodul (10) eingerichtet ist, die Blickrichtung eines Auges (2) des Patienten (20) jeweils basierend auf dem reflektierten Referenzmuster zu bestimmen.

9. Vorrichtung (1) nach einem der Ansprüche 1 bis 8, **dadurch gekennzeichnet, dass** das Verarbeitungsmodul (10) eingerichtet ist, beim Anzeigen der Testmuster jeweils abhängig von den Augenbewegungen eine Reaktionszeit des Patienten (20) zu erfassen, wobei eine Zeitdauer von der Darstellung des Testmusters bis zu einer korrelierenden Augenbewegung des Patienten (20) in Richtung eines Lichtstimulus im betreffenden Testmuster ermittelt wird, und im Gesichtsfeld des Patienten (20) ortsabhängige Reaktionszeiten zu speichern, wobei die Reaktionszeiten jeweils der Position des betreffenden Lichtstimulus zugeordnet gespeichert werden.

10. Vorrichtung (1) nach einem der Ansprüche 1 bis 9, **dadurch gekennzeichnet, dass** das Augenüberwachungsmodul (13) auf einer bei der Gesichtsfeldbestimmung vom Patienten (20) abgewandten Seite des Spiegels (12) angeordnet ist, und dass der Spiegel (12) eine dem Augenüberwachungsmodul (13) vorgelagerte, für mindestens gewisse Wellenlängen lichtdurchlässige Stelle zum Bestimmen der Augenbewegungen aufweist.

11. Vorrichtung (1) nach einem der Ansprüche 1 bis 9, **dadurch gekennzeichnet, dass** das Augenüberwachungsmodul (13) auf einer bei der Gesichtsfeldbestimmung dem Patienten (20) zugewandten Seite des Spiegels (12) angeordnet ist, und dass das Augenüberwachungsmodul (13) eingerichtet ist, die Augenbewegungen via den Spiegel (12) zu bestimmen.

12. Vorrichtung (1) nach einem der Ansprüche 1 bis 9, **dadurch gekennzeichnet, dass** das Augenüberwachungsmodul (13) zur Gesichtsfeldbestimmung mit direkter Sichtlinie auf die Augen (2) des Patienten (20) ausserhalb einer spiegelnden Oberfläche (120) des Spiegels (12) angeordnet ist.

13. Vorrichtung (1) nach einem der Ansprüche 1 bis 12, **dadurch gekennzeichnet, dass** das Augenüberwachungsmodul (13) zwei Bildsensoren umfasst und eingerichtet ist, die Augenbewegungen eines Auges (2) stereoskopisch zu ermitteln.

14. Vorrichtung (1) nach einem der Ansprüche 1 bis 13, **dadurch gekennzeichnet, dass** der Spiegel (12) zur Feldwinkelvergrösserung mit einer nicht-planen Spiegeloberfläche (120) ausgestaltet ist.

15. Verfahren zur Gesichtsfeldbestimmung, umfassend:
Anzeigen (S6) von Testmustern auf einem Bildschirm (11) mit einer Bildschirmanzeigefläche (110),
Erfassen (S7) von Augenbewegungen eines Patienten (20),
Bestimmen (S8) von Blickrichtungen des Patienten (20) beim Anzeigen der Testmuster basierend auf den Augenbewegungen, und
Ermitteln des Gesichtsfelds des Patienten (20) basierend auf den für die Testmuster bestimmten Blickrichtungen,
**gekennzeichnet durch**
Umlenken der Testmuster vom Bildschirm (11) zu den Augen (2) über einen zur Gesichtsfeldbestimmung parallel zur Bipupillarlinie (b) des Patienten (20) ausgerichteten Spiegel (12),
Bestimmen (S8) der relativen Distanz der Augen (2) zum Spiegel (12) respektive zur Bildschirmanzeigefläche (110) basierend auf von einem Augenüberwachungsmodul (13) erfassten Bilddaten oder mittels eines Abstandssensors (18), und
Ermitteln des Gesichtsfelds des Patienten (20) abhängig von der relativen Distanz der Augen (2).

## Claims

1. Device (1) for determining the visual field, comprising:
a screen (11) having a screen display area (110) for displaying test patterns, an eye monitoring module (13) for capturing eye movements of a patient (20), and
a processing module (10) designed to determine viewing directions of the patient (20) on the basis of the eye movements when the test patterns are displayed and establish the visual field of the patient (20) on the basis of the viewing directions determined for the test patterns,
**characterized by**
a mirror (12), which is aligned parallel to the bipupillary line (b) of the patient (20) for determining the visual field, for deflecting the test patterns from the screen (11) to the eyes (2), and
in that the device (1) comprises a distance sensor (18) for determining the relative distance between the eyes (2) and the mirror (12) or the screen display area (110) and in that the processing module (10) is designed to establish the visual field of the patient (20) depending on the relative distance of the eyes (2).

2. Device (1) according to Claim 1, **characterized in that** the screen (11) for determining the visual field can be positioned lying on a horizontal support surface (3), with the screen display area (110) aligned substantially horizontally and facing away from the support surface (3).

3. Device (1) according to one of Claims 1 and 2, **characterized in that** the screen (11) and the mirror (12) are coupled, rotatable about an axis (r), along one of their longitudinal sides such that a mirroring surface (120) of the mirror (12) and a screen display area (110) of the screen (11) can, whilst facing one another, be folded open or shut.

4. Device (1) according to one of Claims 1 to 3, **characterized by** stray-light protection with two stray-light protective screens (14) that are respectively aligned substantially perpendicular to the axis (r) and connected to the screen (11) and the mirror (1 2) in an edge region of the screen (11) and the mirror (12), and arranged in an aperture angle (α) between the screen (11) and the mirror (12).

5. Device (1) according to one of Claims 1 to 4, **characterized in that** the device (1) comprises a sight screen (17) that can be positioned between the screen display area (110) and the patient for determining the visual field such that the screen display area (110) is not directly visible to the patient (20).

6. Device (1) according to one of Claims 1 to 5, **characterized in that** the processing module (10) is designed to display a sequence of test patterns on the screen (11), respectively determine the viewing direction of an eye (2) of the patient (20) when the test patterns are displayed and store said viewing direction assigned to the respective test pattern, and establish the visual field of the patient (20) for one or both eyes (2) on the basis of the viewing directions captured for the sequence of test patterns.

7. Device (1) according to Claim 6, **characterized in that** the processing module (10) is designed to dynamically adapt the sequence of test patterns depending on viewing directions captured for the patient (20).

8. Device (1) according to one of Claims 1 to 7, **characterized in that** the processing module (10) is designed to display one or more reference patterns on the screen (11), **in that** the eye monitoring module (13) is designed to capture reference patterns reflected from an eye of the patient (20) and **in that** the processing module (10) is designed to determine the viewing direction of an eye (2) of the patient (20), respectively based on the reflected reference pattern.

9. Device (1) according to one of Claims 1 to 8, **characterized in that** the processing module (10) is designed to capture a reaction time of the patient (20), respectively depending on the eye movements, when the test patterns are displayed, a period of time being established between displaying the test pattern and a correlated eye movement of the patient (20) in the direction of a light stimulus in the relevant test pattern and to store spatially dependent reaction times in the visual field of the patient (20), the reaction times being respectively stored associated with the position of the relevant light stimulus.

10. Device (1) according to one of Claims 1 to 9, **characterized in that** the eye monitoring module (13) is arranged on a side of the mirror (12) facing away from the patient (20) during the visual field test, and **in that** the mirror (12) has a point, placed in front of the eye monitoring module (13) and permeable to light at at least specific wavelengths, for determining the eye movements.

11. Device (1) according to one of Claims 1 to 9, **characterized in that** the eye monitoring module (13) is arranged on a side of the mirror (12) facing toward the patient (20) during the visual field test, and **in that** the eye monitoring module (13) is designed to determine the eye movements via the mirror (12).

12. Device (1) according to one of Claims 1 to 9, **characterized in that** the eye monitoring module (13), for determining the visual field, is arranged with a direct line of sight to the eyes (2) of the patient (20) and outside of a mirroring surface (120) of the mirror (12).

13. Device (1) according to one of Claims 1 to 12, **characterized in that** the eye monitoring module (13) comprises two image sensors and is designed to establish the eye movements of an eye (2) in a stereoscopic fashion.

14. Device (1) according to one of Claims 1 to 13, **characterized in that** the mirror (12) is embodied with a non-planar mirror surface (120) for increasing the field angle.

15. Method for determining the visual field, comprising the steps of:
displaying (56) test patterns on a screen (11) having a screen display area (110),
capturing (S7) eye movements of a patient (20),
determining (S8) viewing directions of the patient (20) on the basis of the eye movements when the test patterns are displayed, and
establishing the visual field of the patient (20) on the basis of the viewing directions determined for the test patterns,
**characterized by**
deflecting the test patterns from the screen (11) to the eyes (2) via a mirror (12) aligned parallel to the bipupillary line (b) of the patient (20) for determining the visual field,
determining (S8) the relative distance between the eyes (2) and the mirror (12) or the screen display area (110) on the basis of image data captured by an eye monitoring module (13) or by means of a distance sensor (18), and
establishing the visual field of the patient (20) depending on the relative distance of the eyes (2).

## Revendications

1. Dispositif (1) destiné à la détermination du champ visuel, comportant :
un écran (11) avec une surface d'affichage de l'écran (110) pour l'affichage de motifs de test,
un module de surveillance oculaire (13) pour la saisie de mouvements oculaires d'un patient (20), et
un module de traitement (10), installé pour déterminer lors de l'affichage des motifs de test, les directions du regard du patient (20) basées sur les mouvements oculaires, et pour établir le champ visuel du patient (20) basé sur les directions du regard déterminées pour les motifs de test,
**caractérisé par** un miroir (12) orienté vers la détermination du champ visuel parallèle à la ligne bipupillaire (b) du patient (20) pour dévier les motifs de test depuis l'écran (11) vers les yeux (2), et
en ce que le dispositif (1) comporte un capteur de distance (18) pour la détermination de la distance relative des yeux (2) jusqu'au miroir (12) respectivement jusqu'à la surface d'affichage de l'écran (110), et
en ce que le module de traitement (10) est installé pour établir le champ visuel du patient (20) en fonction de la distance relative des yeux (2).

2. Dispositif (1) selon la revendication 1, **caractérisé en ce que** l'écran (11) pour la détermination du champ visuel, reposant sur une surface portante horizontale (3), peut être positionné avec la surface d'affichage de l'écran (110) éloignée de la surface portante (3), orientée pour l'essentiel à l'horizontale.

3. Dispositif (1) selon une des revendications 1 ou 2, **caractérisé en ce que** l'écran (11) et le miroir (12) sont accouplés par un de leur long côté de manière à pouvoir pivoter autour d'un axe (r) de telle sorte qu'une surface réfléchissante (120) du miroir (12) et une surface d'affichage de l'écran (110) de l'écran (11) puissent être séparées respectivement repliées l'une vers l'autre.

4. Dispositif (1) selon une des revendications 1 à 3, **caractérisé par** une protection contre la lumière diffuse avec deux écrans de protection contre la lumière diffuse (14) orientés respectivement pour l'essentiel perpendiculairement à l'axe (r) sur une périphérie de l'écran (11) et du miroir (12) et reliés à l'écran (11) et au miroir (12), et disposés entre l'écran (11) et le miroir (12) avec un angle d'ouverture (α).

5. Dispositif (1) selon une des revendications 1 à 4, **caractérisé en ce que** le dispositif (1) comporte un écran visuel (17) pouvant être positionné, pour la détermination du champ visuel, entre la surface d'affichage de l'écran (110) et le patient de telle sorte que la surface d'affichage de l'écran (110) ne soit pas directement visible par le patient (20).

6. Dispositif (1) selon une des revendications 1 à 5, **caractérisé en ce que** le module de traitement (10) est installé pour afficher une séquence de motifs de test sur l'écran (11), pour déterminer respectivement la direction du regard d'un oeil (2) du patient (20) lors de l'affichage des motifs de test et pour la stocker en l'attribuant au motif de test concerné, et pour établir le champ visuel du patient (20) basé sur un oeil ou sur les deux yeux (2) pour les directions du regard saisies pour la séquence de motifs de test.

7. Dispositif (1) selon la revendication 6, **caractérisé en ce que** le module de traitement (10) est installé pour adapter dynamiquement la séquence des motifs de test en fonction de directions du regard saisies pour le patient (20).

8. Dispositif (1) selon une des revendications 1 à 7, **caractérisé en ce que** le module de traitement (10) est installé pour afficher un ou plusieurs motifs de référence sur l'écran (11), **en ce que** le module de surveillance oculaire (13) est installé pour saisir des motifs de référence réfléchis par un oeil du patient (20), et **en ce que** le module de traitement (10) est installé pour déterminer la direction du regard d'un oeil (2) du patient (20) respectivement basé sur le motif de référence réfléchi.

9. Dispositif (1) selon une des revendications 1 à 8, **caractérisé en ce que** le module de traitement (10) est installé pour saisir, lors de l'affichage des motifs de test, un temps de réaction du patient (20) respectivement en fonction des mouvements oculaires, une durée de la représentation du motifs de test jusqu'à un mouvement oculaire corrélé du patient (20) en direction d'un stimulus lumineux dans le motif de test concerné étant déterminée, et pour stocker des temps de réaction en fonction de la localisation dans le champ visuel du patient (20), les temps de réaction attribués respectivement à la position du stimulus lumineux concerné étant stockés.

10. Dispositif (1) selon une des revendications 1 à 9, **caractérisé en ce que** le module de surveillance oculaire (13) est disposé sur une face du miroir (12) opposée au patient (20) lors de la détermination du champ visuel, et **en ce que** le miroir (12) présente en avant du module de surveillance oculaire (13), un endroit transparent à la lumière pour au moins certaines longueurs d'ondes pour déterminer les mouvements oculaires.

11. Dispositif (1) selon une des revendications 1 à 9, **caractérisé en ce que** le module de surveillance oculaire (13) est disposé sur une face du miroir (12) dirigée vers le patient (20) lors de la détermination du champ visuel, et **en ce que** le module de surveillance oculaire (13) est installé pour déterminer les mouvements oculaires via le miroir (12).

12. Dispositif (1) selon une des revendications 1 à 9, **caractérisé en ce que** le module de surveillance oculaire (13) pour la détermination du champ visuel est disposé en vue directe vers les yeux (2) du patient (20) à l'extérieur d'une surface réfléchissante (120) du miroir (12).

13. Dispositif (1) selon une des revendications 1 à 12, **caractérisé en ce que** le module de surveillance oculaire (13) comporte deux capteurs d'images et qu'il est installé pour déterminer les mouvements oculaires d'un oeil (2) de manière stéréoscopique.

14. Dispositif (1) selon une des revendications 1 à 13, **caractérisé en ce que** le miroir (12) est pourvu d'une surface réfléchissante (120) non plane pour augmenter l'angle du champ.

15. Procédé destiné à la détermination du champ visuel, comportant :
l'affichage (S6) de motifs de test sur un écran (11) avec une surface d'affichage de l'écran (110),
la saisie (S7) de mouvements oculaires d'un patient (20),
la détermination (58) de directions du regard du patient (20) lors de l'affichage des motifs de test, basée sur les mouvements oculaires, et
la détermination du champ visuel du patient (20) basé sur les directions du regard déterminées pour les motifs de test,
**caractérisé par**
la déviation des motifs de test depuis l'écran (11) vers les yeux (2) par l'intermédiaire d'un miroir (12) orienté parallèlement à la ligne bipupillaire (b) du patient (20),
la détermination (S8) de la distance relative des yeux (2) jusqu'au miroir (12) respectivement jusqu'à la surface d'affichage de l'écran (110) basée sur des données d'images saisies par un module de surveillance oculaire (13) ou au moyen d'un capteur de distance (18), et
l'établissement du champ visuel du patient (20) en fonction de la distance relative des yeux (2).
